# EUROPEAN PATENT SPECIFICATION

(11) **EP 4 574 195 B1**
(45) Date of publication and mention of the grant of the patent: **02.12.2026**
(21) Application number: 25161660.3
(22) Date of filing: 17.09.2020
(51) Int. Cl.: A61B 17/88, A61B 17/00, A61B 17/34, A61M 25/01, A61M 25/10

(54) **OSTEOTOME WITH INFLATABLE PORTION AND MULTIWIRE ARTICULATION**
OSTEOTOM MIT AUFBLASBAREM TEIL UND MEHRDRAHTGELENK
OSTÉOTOME AVEC PARTIE GONFLABLE ET ARTICULATION À FILS MULTIPLES

(30) Priority: 18.09.2019 US 201962902144 P
(43) Date of publication of application: 25.06.2025
(62) Document number(s) of the earlier application(s) in accordance with Art. 76 EPC: 20865049.9 / 4 031 040
(73) Proprietor: MERIT MEDICAL SYSTEMS, INC., South Jordan, UT 84095 (US)
(72) Inventor: WIERSDORF, Jason, West Jordan, Utah 84081 (US); HOPKINSON, Aaron, Herriman, Utah 84098 (US)
(74) Representative: Cleveland Scott York

(56) References cited:
- EP-A1- 3 260 069
- WO-A1-2009/065085
- US-A1- 2011 190 831
- US-A1- 2013 197 563
- US-A1- 2014 357 983
- US-A1- 2016 120 584

## Description

### RELATED APPLICATIONS

This application claims priority to United States Provisional Patent Application No. 62/902,144, filed on September 18, 2019 and titled, "Osteotome with Inflatable Portion and Multiwire Articulation,".

### TECHNICAL FIELD

The present disclosure relates generally to medical devices used to treat tissue, including bone. More specifically, in certain embodiments, the present disclosure relates to medical devices used to displace tissue using an expandable member, such as a balloon.

### BRIEF DESCRIPTION OF PRIOR ART

US2011/190831 relates to a system and method for performing a surgical procedure, and in particular, to an articulating balloon catheter.

### BRIEF DESCRIPTION OF THE DRAWINGS

The embodiments disclosed herein will become more fully apparent from the following description and appended claims, taken in conjunction with the accompanying drawings. These drawings depict only typical embodiments, which will be described with additional specificity and detail through use of the accompanying drawings in which:
FIG. 1A is a side view of an embodiment of a bone displacement device in a ready state.
FIG. 1B is a side view of the bone displacement device of FIG. 1A in a bone displacement state.
FIG. 2 is a longitudinal cross-sectional view of a proximal portion of the bone displacement device of FIG. 1A.
FIG. 3 is a transverse cross-sectional view of a middle portion of the bone displacement device of FIG. 1A.
FIG. 4A is a longitudinal cross-sectional view of a distal portion of the bone displacement device of FIG. 1A in an "expandable member expanded" state.
FIG. 4B is a longitudinal cross-sectional view of the distal portion of the bone displacement device of FIG. 4A, in an "expandable member not expanded" state.
FIG. 5A is a side view of another embodiment of a bone displacement device in a ready state.
FIG. 5B is a side view of the bone displacement device of FIG. 5A in a bone displacement state.
FIG. 6 is a longitudinal cross-sectional view of a proximal portion of the bone displacement device of FIG. 5A.
FIG. 7 is a transverse cross-sectional view of a middle portion of the bone displacement device of FIG. 5A.
FIG. 8A is a longitudinal cross-sectional view of a distal portion of the bone displacement device of FIG. 5A in an "expandable member expanded" state.
FIG. 8B is a longitudinal cross-sectional view of the distal portion of the bone displacement device of FIG. 8A, in an "expandable member not expanded" state.
FIG. 9A is a side view of another embodiment of a bone displacement device in a ready state.
FIG. 9B is a side view of the bone displacement device of FIG. 9A in a bone displacement state.
FIG. 10 is a cross-sectional view of a proximal portion of the bone displacement device of FIG. 9A.
FIG. 11 is a side view of a stylet of the bone displacement device of FIG. 9A.
FIG. 12 is a transverse cross-sectional view of a middle portion of the bone displacement device of FIG. 9A.
FIG. 13A is a longitudinal cross-sectional view of a distal portion of the bone displacement device of FIG. 9A in an "expandable member expanded" state.
FIG. 13B is a longitudinal cross-sectional view of the distal portion of the bone displacement device of FIG. 9A, in an "expandable member not expanded" state.
FIG. 14A is a side view of another embodiment of a bone displacement device in a package state.
FIG. 14B is a side view of the bone displacement device of FIG. 14A in a ready state.

### SUMMARY OF THE INVENTION

The invention is set out in the appended set of claims.

### DETAILED DESCRIPTION

According to the present invention, a bone displacement system comprises:
a bone displacement device, comprising:an elongate outer tube;
an elongate stylet coaxially disposed within the outer tube, wherein the stylet comprises a tubular shaft with a shaft lumen;
a handle coupled to a proximal portion of the outer tube and the stylet; and a balloon disposed adjacent a distal end of the outer tube; and
a tip tie tube coupled to a distal end of the balloon and slidably disposed over a distal portion of stylet,
wherein the elongate stylet extend partially into the balloon and partially into the tip tie tube; a protective sleeve configured to be disposed around the balloon.
In general, tissue treatment devices may include elongate members, expandable members, and other components. In some instances, an elongate member of a tissue treatment device may be advanced to a treatment location and an expandable member expanded to displace tissue. For example, a bone displacement device may be disposed within a vertebra of a patient and an expandable member expanded to displace portions of the vertebra. Similarly, such devices may be utilized in other areas of the body with other types of tissue. For convenience, including when describing the illustrated embodiments, this disclosure references "bone displacement" or "bone displacement devices," however, such disclosure may be analogously applied to devices, elements, and procedures configured to displace or otherwise treat tissue in other portions of the body.

For example, a stylet may comprise a plurality of pull wires coaxially disposed within a shaft of the stylet. A distal portion of the stylet may be articulated when a tension force is applied to the plurality of pull wires. In some embodiments, a portion of the handle is configured as an actuator to articulate the stylet.

For example, in some embodiments, the handle comprises a rotatable grip having female threads configured to engage with male threads of a pull member. The threads may include thread stops to limit rotation of the rotatable grip. A distal portion of the plurality of pull wires is coupled to the pull member. In such embodiments, rotation of the rotatable grip in the first direction proximally displaces the pull member and thus applies a tension force to the pull wires to articulate the distal portion of the stylet. The handle may also include a side port having a valve.

According to the invention, the expandable member is a balloon. The expandable member may be disposed at a distal portion of the bone displacement device. In some embodiments, a proximal portion of the expandable member is attached to a distal portion of the outer tube via a tie layer. A distal portion of the expandable member can be attached to a tip tie tube. The tip tie tube may be longitudinally displaceable over the distal portion of the stylet. A protective sleeve may be disposed around the balloon when the bone displacement device is in its package, and displaced proximally over the outer tube to engage with the handle when the bone displacement device is ready to use.

In certain instances, a bone displacement device may be used by a practitioner to treat a fractured bone, such as a vertebral bone. The practitioner may displace bone by inflating an expandable member at a distal end of the bone displacement device to create a cavity into which a bone stabilizing material, such as bone cement, may be injected. The rotatable grip may be rotated in a first direction to apply a tension force to pull wires to articulate a distal portion of the bone displacement device. The distal portion of the bone displacement device may be directed - via articulation of the distal portion and/or displacement of the entire bone displacement device - to a desired location within the bone. The expandable member can be expanded to displace bone tissue adjacent the expandable member to create a cavity. The bone displacement device can be removed from the bone to allow for injection of bone cement into the cavity.

Embodiments may be understood by reference to the drawings, wherein like parts are designated by like numerals throughout.

The phrases "coupled to" and "in communication with" refer to any form of interaction between two or more entities, including mechanical, electrical, magnetic, electromagnetic, fluid, and thermal interaction. Two components may be coupled to or in communication with each other even though they are not in direct contact with each other. For example, two components may be coupled to or in communication with each other through an intermediate component.

The directional terms "distal" and "proximal" are given their ordinary meaning in the art. That is, the distal end of a medical device means the end of the device furthest from the practitioner during use. The proximal end refers to the opposite end, or the end nearest the practitioner during use. As specifically applied to a bone displacement device, the proximal end of the device refers to the end nearest the handle and the distal end refers to the opposite end, the end nearest a working tip of the device. If at one or more points in a procedure a physician changes the orientation of a bone displacement device, as used herein, the term "proximal end" always refers to the handle end of the device (even if the distal end is temporarily closer to the physician).

"Fluid" is used in its broadest sense, to refer to any fluid, including both liquids and gases as well as solutions, compounds, suspensions, etc., which generally behave as fluids.

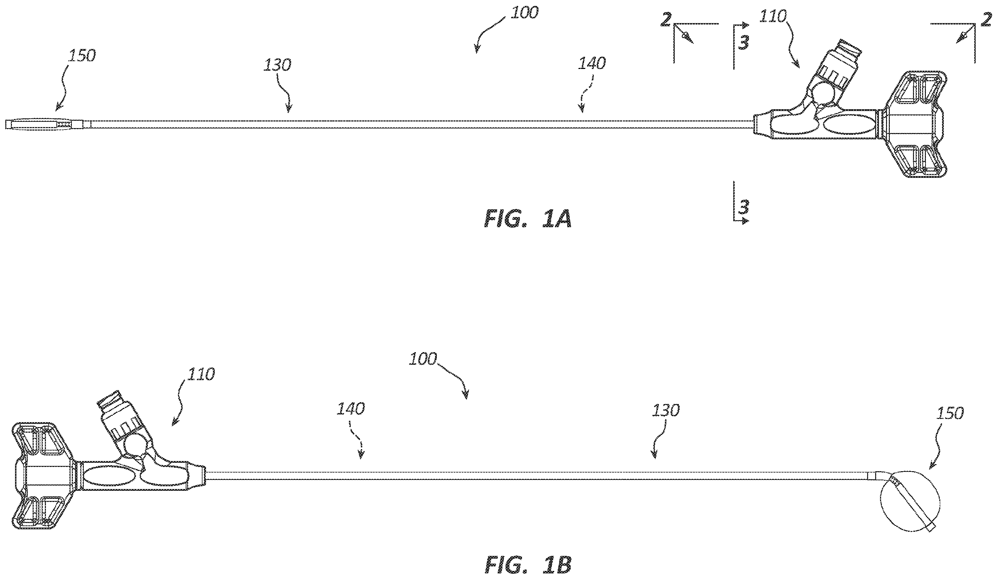

FIGS. 1A-14B illustrate different views of several bone displacement devices or osteotomes and related components. In certain views each device may be coupled to, or shown with, additional components not included in every view. Further, in some views only selected components are illustrated, to provide detail into the relationship of the components. Some components may be shown in multiple views, but not discussed in connection with every view. Disclosure provided in connection with any figure is relevant and applicable to disclosure provided in connection with any other figure or embodiment.

FIGS. 1A-4B depict an embodiment of a bone displacement device 100. In the illustrated embodiment, the bone displacement device 100 comprises a handle 110, a catheter 130, a stylet 140, and an expandable member 150. FIG. 1A shows the bone displacement device 100 in a ready state where the expandable member 150 is not expanded and a distal portion of the bone displacement device 100 is not articulated. FIG. 1B shows the bone displacement device 100 in a bone displacement or cavity forming state where the expandable member 150 is expanded and the distal portion is articulated.

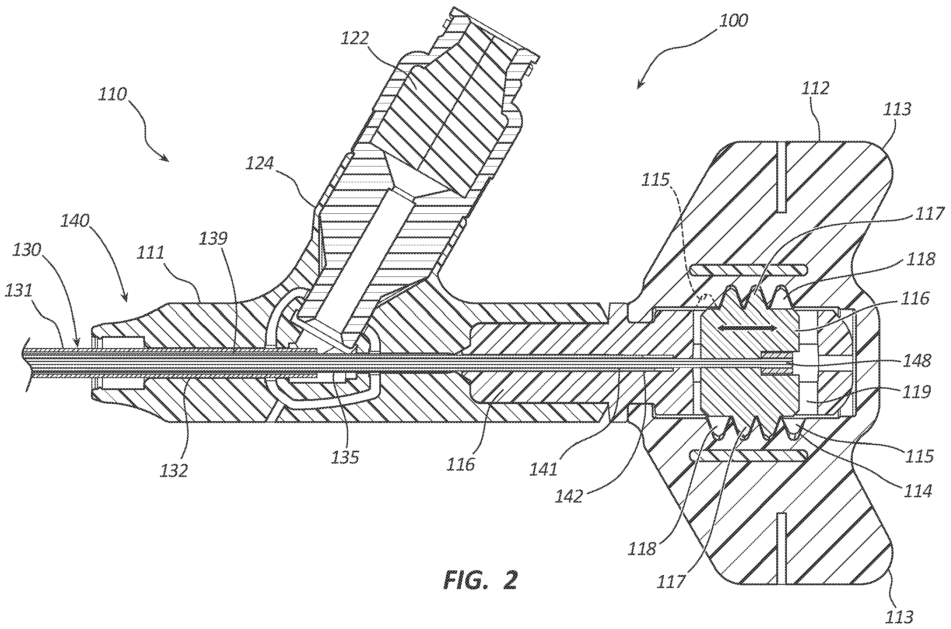

FIG. 2 illustrates a proximal portion of the bone displacement device 100. The proximal portion comprises the handle 110, a proximal portion of the catheter 130, and a proximal portion of the stylet 140. The catheter 130 may comprise an elongate outer tube 131 and an elongate inner tube 135. The outer tube 131 comprises a proximal portion 132. The outer tube 131 can be formed from any suitable polymeric material. For example, the outer tube 131 may be formed from polyurethane, nylon, PBT, polyethylene, polypropylene, etc. A proximal end of outer tube 131 can be fixedly coupled to the handle 110. In some embodiments, the inner tube 135 is coaxially disposed within the outer tube 131. The inner tube 135 may be formed from any of the materials listed in connection with the outer tube 131, though the inner tube 135 and outer tube 131 may or may not be formed of the same material. A proximal end of the inner tube 135 can be fixedly coupled to the handle 110 at a location proximal of the proximal end of the outer tube 131.

In the illustrated embodiment, the stylet 140 is shown to comprise a tubular shaft 141. The shaft 141 may be formed from any suitable rigid material, such as stainless steel, titanium, nitinol, etc. A proximal end of the shaft 141 may be fixedly coupled to the handle 110 at a location proximal of the proximal end of the inner tube 135. One or more pull wires 142 can be coaxially disposed within the shaft 141. In the illustrated embodiment, the pull wires 142 include a plurality of pull wires comprising seven pull wires. In other embodiments, the number of pull wires 142 may be three, four, five, six, eight, or more pull wires. The pull wires 142 may extend proximally from the shaft 141 and be fixedly coupled to the distal end of the shaft 141. The pull wires 142 may be formed from any suitable material with high tensile strength. For example, the pull wires 142 may be formed from stainless steel, titanium, nitinol, etc. A diameter of the pull wires 142 may range from about 0.005 inch (0.127mm) to about 0.030 inch (0.762 mm).

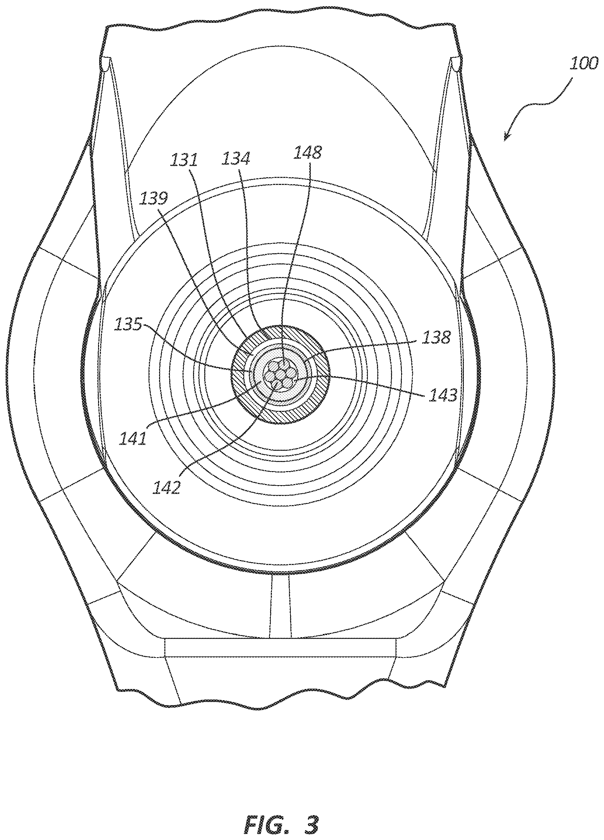

FIG. 3 illustrates a transverse cross-section of a middle portion of the bone displacement device 100. As illustrated, the outer tube 131 includes an outer lumen 134. The inner tube 135 is shown to be coaxially disposed within the outer lumen 134. An annular space 139 may be formed between the outer tube 131 and the inner tube 135. The inner tube 135 may include an inner lumen 138. The shaft 141 may be coaxially disposed within the inner lumen 138. The shaft 141 is shown to include a shaft lumen 143. The pull wires 142 are disposed within the shaft lumen 143. As depicted in FIG. 3, the pull wires 142 collectively include seven individual pull wires 148. The individual pull wires 148 are disposed with a first individual pull wire 148 centrally located and the remaining six individual pull wires 148 equally spaced around the first individual pull wire 148. The pull wires 148 are shown as individual strands disposed longitudinally adjacent each other (without braiding or twisting) in the illustrated embodiment. This configuration of the pull wires 148 may be configured to provide adequate stiffness to the bone displacement device 100 to the ready state where the distal portion of the bone displacement device 100 is not articulated and/or to facilitate transfer of force to straighten the bone displacement device 100 from an articulated configuration to a non articulated configuration. In another embodiment, each pull wire 148 may be formed in a braid of a plurality of smaller diameter wires. Other arrangements of the pull wires 148 are likewise within the scope of this disclosure, including embodiments where the individual wires are braided or twisted into a cable.

Referring again to FIG. 2, the handle 110 may comprise a body 111 and a rotatable grip 112 rotatably coupled to a proximal end of the body 111. The body 111 and rotatable grip 112 may be formed from any suitable polymeric material, such as polycarbonate, acrylonitrile butadiene styrene, etc. The body 111 may comprise a side port 124. In the illustrated embodiment, the side port 124 extends generally proximally and laterally from a longitudinal axis of the body 111. The side port 124 may extend at an angle ranging from 15 degrees to 90 degrees or about 45 degrees relative to the longitudinal axis. The side port 124 may be in fluid communication with the annular space (139 of FIG. 3.) During some therapies, a fluid delivery device (e.g., syringe) can be releasably coupled to a proximal portion of the side port 124. A valve member 122 may be disposed within the side port 124. The valve member 122 may be configured to selectively permit air or fluid to be directed from the fluid delivery device, through the side port 124, through the annular space 139, and then to the expandable member (150 of FIG. 1A) when the fluid delivery device is coupled to the side port 124. The valve member 122 may also be configured to retain the air or fluid within the annular space 139 and the expandable member 150 when the fluid delivery device is removed from the side port 124.

In the illustrated embodiment, the proximal end of the outer tube 131 is coupled to the body 111 at a location distal to the side port 124, and the inner tube 135 is coupled to the body 111 at a location proximal to the side port 124. Further, a proximal end of the shaft 141 can be coupled to the body 111 at a location proximal to the inner tube 135.

The rotatable grip 112 may be configured to be rotated around a longitudinal axis of the handle 110. As depicted, the rotatable grip 112 may comprise laterally extending wings 113 configured to be gripped by a user. In other embodiments, the rotatable grip 112 may comprise any suitable feature to facilitate gripping and rotation of the rotatable grip 112. For example, the rotatable grip 112 may comprise a knob including grippable features, such as ridges, bumps, recesses, textured surface, etc. The rotatable grip 112 may comprise a chamber 119 configured to receive a pull member 116. As shown in the figures, and discussed below, the pull member 116 of the illustrated embodiment is configured as non-rotatable relative to the rotatable grip 112, or configured not to rotate with the rotatable grip 112. The chamber 119 may include a female thread 114 configured to engage a male thread 117 of the pull member 116. The female thread 114 may comprise proximal and distal female thread stops 115. In the illustrated embodiment, the female thread stops 115 may include a flat face oriented perpendicular to a longitudinal axis of the rotatable grip 112. In another embodiment, the female thread stops 115 may include a tapered face. The pull member 116 may include a male thread 117 configured to engage with the female thread 114. The male thread 117 may comprise proximal and distal male thread stops 118. In the illustrated embodiment, the male thread stops 118 may include a flat face oriented perpendicular to a longitudinal axis of the pull member 116. In another embodiment, the male thread stops 118 may include a tapered face. The female thread stops 115 can be configured to engage with the male thread stops 118 to prevent over-rotation of the rotatable grip 112 in both a first direction and a second direction. In some instances, over-rotation of the rotatable grip 112 may result in excess strain or breakage of the shaft 141 and/or one or more of the individual pull wires 148. Thus, the threads may be configured with a positive stop to minimize excess force and subsequent breakage. In another embodiment, the chamber 119 may include a male thread 117 and male thread stops 118 while the pull member 116 includes a female thread 114 and female thread stops 115.

In the illustrated embodiment, the proximal ends of the pull wires 142 may be fixedly coupled to the pull member 116. When the rotatable grip 112 is rotated in the first direction, the pull member 116 is displaced proximally, causing a tension force to be equally or substantially equally applied to all of the individual pull wires 148. Embodiments wherein one or more individual pull wires 148 transmit an uneven or larger portion of the tension force are likewise within the scope of this disclosure. The tension force on the pull wires 142 may result in a tension force being applied to the distal end of the stylet 140 and articulation of the distal portion of the bone displacement device 100, as shown in FIG. 1B. During actuation, the rotatable grip 112 may be rotated from about one degree to about 540 degrees, from about one degree to about 360 degrees, or from about one degree to about 180 degrees, or along any portion of these ranges. Rotation of the rotatable grip 112 in the first direction may be stopped when the proximal female thread stop 115 engages with the proximal male thread stop 118, or may be stopped at any point along the range of rotation of the rotatable grip 112, meaning it is stopped by a user at a point of partial rotation along the rotational range of the rotatable grip 112. Rotation of the rotatable grip 112 and proximal displacement of the pull member 116 may result in an articulation of the distal portion of the bone displacement device 100 from about zero degrees to about 180 degrees, from about zero degrees to about 135 degrees, or from about zero degrees to about 90 degrees. In some embodiments, the rotatable grip 112 may comprise a rotation lock configured to maintain the rotatable grip 112 in a partial or fully rotated state when the rotatable grip 112 is released by the user. For example, the body 111 may comprise teeth configured to engage with opposing teeth of the rotatable grip 112.

Rotation of the rotatable grip 112 in the second direction may displace the pull member 116 distally and release the tension force on the pull wires 142, resulting in the distal portion of the bone displacement device 100 returning to a straight configuration, as shown in FIG. 1A. The rotatable grip 112 can be rotated in the second direction until the distal female thread stop 115 engages with the distal male thread stop 118.

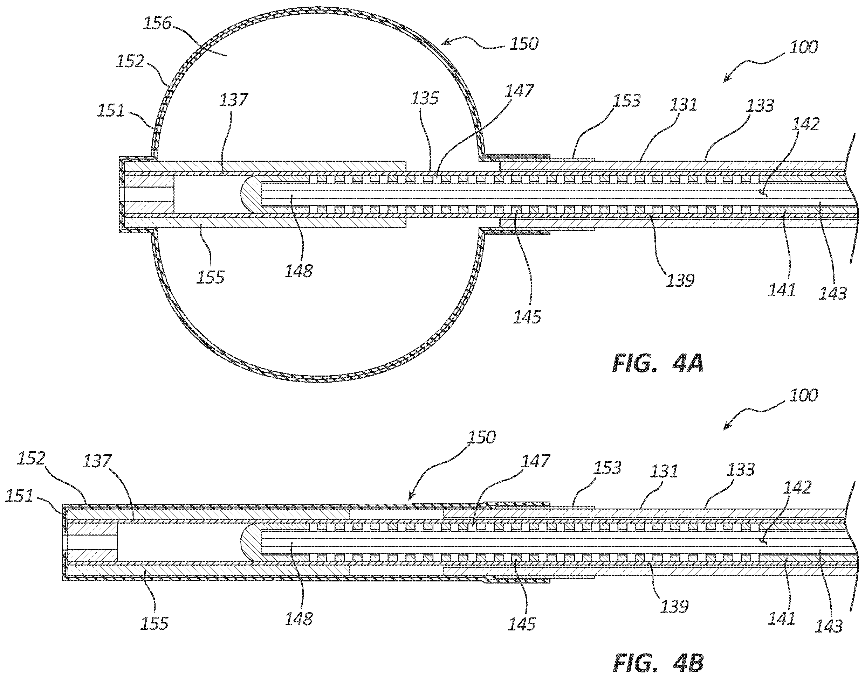

FIGS. 4A-4B illustrate a distal portion of the bone displacement device 100. The distal portion comprises the expandable member 150, a distal portion 133 of the outer tube 131, a distal portion 137 of the inner tube 135, and a distal portion 145 of the shaft 141. The inner tube 135 and the shaft 141 may extend beyond a distal end of the outer tube 131. The inner tube 135 may extend beyond a distal end of the shaft 141. According to the invention, the expandable member 150 comprises a balloon 151. In other examples, the expandable member 150 may comprise any suitable expandable and retractable mechanism. For example, the expandable member 150 may comprise a plurality of ribs configured to expand radially outward.

The balloon 151 may comprise a double balloon wall 152 configured to expand radially outward without stretching when air or fluid is injected into the balloon 151, for example, through the annular space 139. Embodiments wherein the balloon wall comprises more or fewer layers are likewise within the scope of this disclosure. A thickness of the double balloon wall 152 may range from about 0.020 mm to about 0.038 mm or from about .025 mm to about 0.030 mm. A length of the balloon 151 when not inflated or expanded may range from about 10 mm to about 30 mm. The balloon 151 may be formed of any suitable non-compliant polymeric material, such as engineered plastic polyurethane (e.g., Isoplast^{®}), nylon, polybutylene terephthalate, etc. A proximal portion of the balloon 151 may be sealingly coupled to the distal end of the outer tube 131. A tie layer 153 may be disposed between the balloon wall 152 and the outer tube 131 to facilitate bonding of the balloon wall 152 to the outer tube 131. In other embodiments, the tie layer 153 is not used to facilitate bonding of the balloon wall 152 to the outer tube 131. The tie layer 153 may be formed from a polyurethane material having an intermediate hardness that is between the hardness of the material of the balloon 151 and the hardness of the material of the outer tube 131. The balloon wall 152 may be bonded to the tie layer 153 and the outer tube 131 using any suitable technique, such as heat, radio frequency, solvent bonding, gluing, etc.

A distal portion of the balloon 151 may be sealingly coupled to the distal end of the inner tube 135. A tip tie tube 155 may be disposed between the balloon wall 152 and the inner tube 135. The tip tie tube 155 may be formed from a material similar to the tie layer 153. In some embodiments, the tip tie tube 155 may comprise a braided structure. The tip tie tube 155 may extend proximally over the distal portion 137 of the inner tube 135 and the distal portion 145 of the shaft 141. The tip tie tube 155 and shaft 141 are configured in a "piston/cylinder" type arrangements where the shaft 141 is allowed to move with respect to the tip tie tube when the balloon 151 is inflated or deflated. For example, the tip tie tube 155 may be configured to piston proximally over the shaft 141 when the balloon 151 is inflated and to piston distally over the shaft 141 when the balloon 151 is deflated. The tip tie tube 155 may be configured to facilitate bonding of the balloon wall 152 to the inner tube 135. In other embodiments, the tip tie tube 155 may provide structural support to the inner tube 135 to prevent kinking of the inner tube 135 when inserted into a vertebral bone.

The seal of the proximal end of the balloon 151 to the outer tube 131 and the seal of the distal end of the balloon 151 to the inner tube 135 may form a balloon chamber 156 configured to be pressurized. The balloon chamber 156 may be pressurized up to a pressure of about 60 atm. The balloon 151 may be expanded to a diameter of from about 10 mm to about 30 mm.

As shown in FIGS. 4A-4B, the distal portion 145 of the shaft 141 may comprise a plurality of laser cuts 147 (not claimed). The laser cuts 147 may facilitate articulation of the shaft 141 in a single plane when the tension force is applied to the pull wires 142, as shown in FIG 1B. The laser cuts 147 may comprise variables (e.g., depth, pitch, spacing) that control the articulation parameters of the shaft 141, such as angle of articulation. In certain examples, the laser cuts 147 may comprise a tab configured to be received by a recess. The laser cuts 147 of this example may prevent torsional rotation of the laser cuts 147 relative to one another. The pull wires 142 may also prevent torsional rotation of the laser cuts 147 because the pull wires 142 substantially fill the shaft lumen 143 preventing axial misalignment of the laser cuts 147. The laser cuts 147 may extend partially through a diameter of the shaft 141, leaving a spine of uncut material. When articulated, the shaft 141 may bend toward the spine while a distance between shaft segments on either side of the laser cuts 147 increases on an opposing side of the shaft 141, defining a substantially "V" shape.

During use, in some instances, the shaft 141 may break at one of the laser cuts 147 when the shaft 141 is articulated. This may be due to external forces (such as from the bone) acting on the shaft 141. In the event of a breakage, the pull wires 142 may prevent a portion of the shaft 141 distal to the break from breaking away from a remainder of the shaft 141. Even if one of the individual pull wires 148 also breaks, the remaining individual pull wires 148 may retain attachment to the distal end of the shaft 141. Furthermore, embodiments wherein the pull wires 142 collectively fill the shaft lumen 143, even in an event of a break in the shaft 141, the pull wires 142 may maintain the coaxial arrangement of the shaft 141, inner tube 135, and outer tube 131 and prevent leakage of air or fluid from the bone displacement device 100 caused by damage to the inner tube 135 and/or outer tube 131 by a broken end of the shaft 141 of a pull wire 148 .

In use, a bone displacement device may be used to displace bone tissue. A distal end of the bone displacement device may be inserted into a vertebral bone, for example, through an introducer cannula. A distal portion of the bone displacement device may be articulated when a rotatable grip is rotated in a first direction, causing one or more pull members to be displaced proximally. Proximal displacement of the pull member may apply a tension force to pull wires disposed within a stylet shaft and coupled to the pull member. The tension force applied to the pull wires can cause a distal portion of a stylet to articulate the distal portion of the bone displacement device. The articulated bone displacement device can be directed to a desired location within the vertebral bone. A syringe may be coupled to a side port of a handle. Air or fluid may be delivered through the side port to an expandable member (e.g., balloon) disposed adjacent the distal end of the bone displacement device. The air or fluid may expand the expandable member to displace adjacent bone tissue.

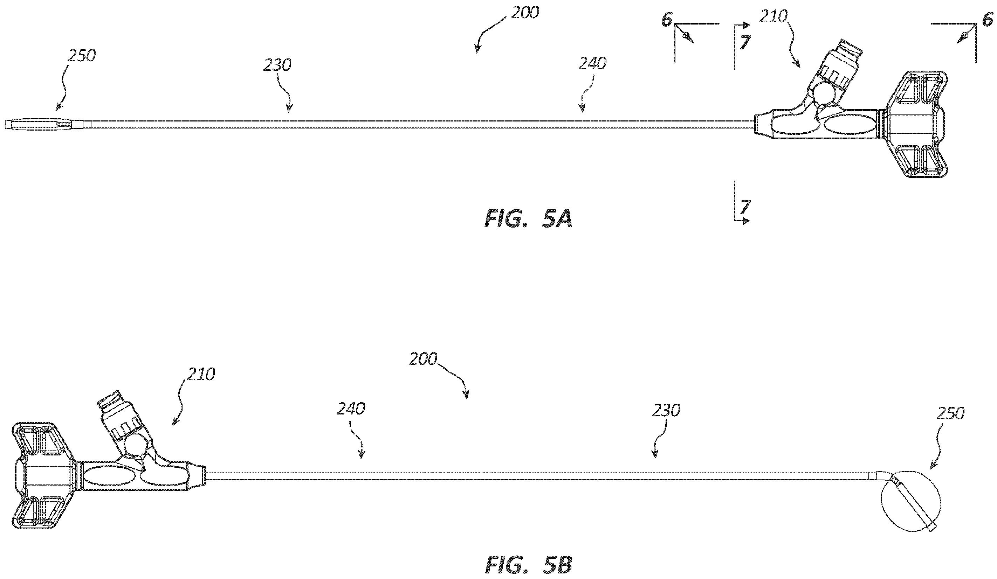

FIGS. 5A-8B depict an embodiment of a bone displacement device 200 that resembles the bone displacement device 100 described above in certain respects. Accordingly, like features are designated with like reference numerals, with the leading digit incremented to "2." For example, the embodiment depicted in FIGS. 5A-8B includes a handle 210 that may, in some respects, resemble the handle 110 of FIG. 1A. Relevant disclosure set forth above regarding similarly identified features thus may not be repeated hereafter. Moreover, specific features of the bone displacement device 100 and related components shown in FIGS. 1A-4A may not be shown or identified by a reference numeral in the drawings or specifically discussed in the written description that follows. However, such features may clearly be the same, or substantially the same, as features depicted in other embodiments and/or described with respect to such embodiments. Accordingly, the relevant descriptions of such features apply equally to the features of the bone displacement device 200 and related components depicted in FIGS. 5A-8B. Any suitable combination of the features, and variations of the same, described with respect to the bone displacement device 100 and related components illustrated in FIGS. 1A-4B can be employed with the bone displacement device 200 and related components of FIGS. 5A-8B, and vice versa. This pattern of disclosure applies equally to further embodiments depicted in subsequent figures and described hereafter, wherein the leading digits may be further incremented.

FIGS. 5A-8B depict another embodiment of a bone displacement device 200. In the illustrated embodiment of FIGS. 5A-8B, the bone displacement device 200 comprises a handle 210, a catheter 230, a stylet 240, and an expandable member 250. FIG. 5A shows the bone displacement device 200 in a ready state where the expandable member 250 is not expanded and a distal portion of the bone displacement device 200 is substantially straight. FIG. 5B shows the bone displacement device 200 in a cavity forming state where the expandable member 250 is expanded and the distal portion is articulated.

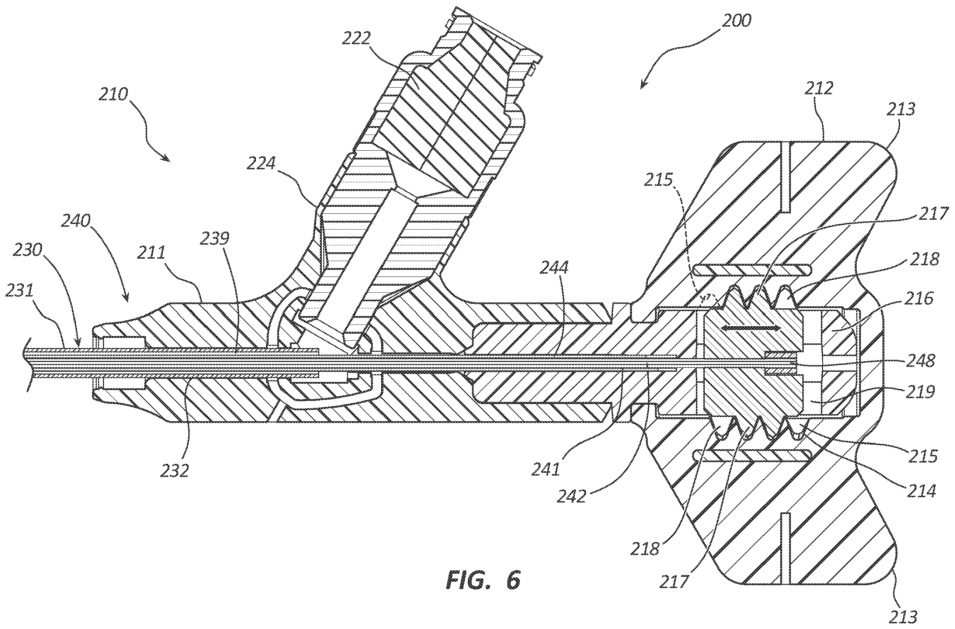

FIG. 6 illustrates a proximal portion of the bone displacement device 200. The proximal portion comprises the handle 210, a proximal portion of the catheter 230, and a proximal portion of the stylet 240. The catheter 230 may comprise an elongate outer tube 231 having a proximal portion 232. A proximal end of the outer tube 231 can be fixedly coupled to the handle 210.

The stylet 240 is shown to comprise a tubular shaft 241 having a proximal portion 244. A proximal end of the shaft 241 may be fixedly coupled to the handle 210 at a location proximal of the proximal end of the outer tube 231. One or more pull wires 242 can be disposed within the shaft 241.

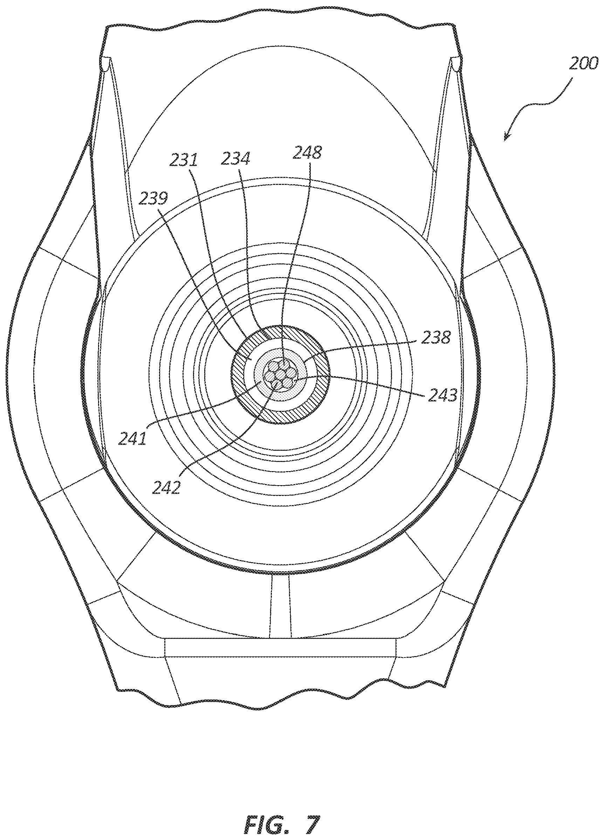

FIG. 7 illustrates a transverse cross-section of a middle portion of the bone displacement device 200. As illustrated, the outer tube 231 includes an outer lumen 234. The shaft 241 is coaxially disposed within the outer lumen 234. An annular space 239 may be formed between the outer tube 231 and the shaft 241. The shaft 241 may include a shaft lumen 243. The pull wires 242 can be disposed within the shaft lumen 243.

Referring again to FIG. 6, the handle 210 may comprise a body 211 and a rotatable grip 212 rotatably coupled to a proximal end of the body 211. The body 211 may comprise a side port 224 extending laterally from a longitudinal axis of the body 211. The side port 224 may be in fluid communication with the annular space (239 of FIG. 7). A fluid delivery device (e.g., syringe) can be releasably coupled to a proximal portion of the side port 224. A valve member 222 may be disposed within the side port 124. The valve member 222 may be configured to selectively permit air or fluid to be directed through the side port 224, through the annular space 239, and to the expandable member 250 when the fluid delivery device is coupled to the side port 224. The valve member 222 may also be configured to retain the air or fluid within the annular space 239 and the expandable member 250 when the fluid delivery device is removed from the side port 224.

In the illustrated embodiment, the proximal end of the outer tube 231 can be coupled to the body 211 at a location distal to the side port 224. The shaft 241 can be coupled to the body 211 at a location proximal to the outer tube 231.

The rotatable grip 212 may be configured to be rotatable around a longitudinal axis of the handle 210. The rotatable grip 212 may comprise generally laterally and proximally extending wings 213 configured to be gripped by a user. The rotatable grip 212 may comprise a chamber 219 configured to receive a pull member 216. The chamber 219 may include a female thread 214 configured to engage a male thread 217 of the pull member 216. The female thread 214 may comprise proximal and distal thread stops 215. The male thread 217 may comprise proximal and distal thread stops 218. The female thread stops 215 can be configured to engage with the male thread stops 218 to prevent over-rotation of the rotatable grip 212 in both a first direction and a second direction.

In the illustrated embodiment, the proximal ends of the pull wires 242 may be fixedly coupled to the pull member 216. When the rotatable grip 212 is rotated in the first direction, the pull member 216 can be displaced proximally, causing a tension force to be applied to the pull wires 242. The tension force on the pull wires 242 may result in a tension force being applied to the distal end of the shaft 241 and articulation of the shaft 241 and the bone displacement device 200, as shown in FIG. 5B.

Rotation of the rotatable grip 212 in the second direction may displace the pull member 216 distally and release the tension force on the pull wires 242, resulting in the distal portion of the bone displacement device 200 returning to a substantially straight configuration, as shown in FIG. 5A. The rotatable grip 212 can be rotated in the second direction until the distal female thread stop 215 engages with the distal male thread stop 218.

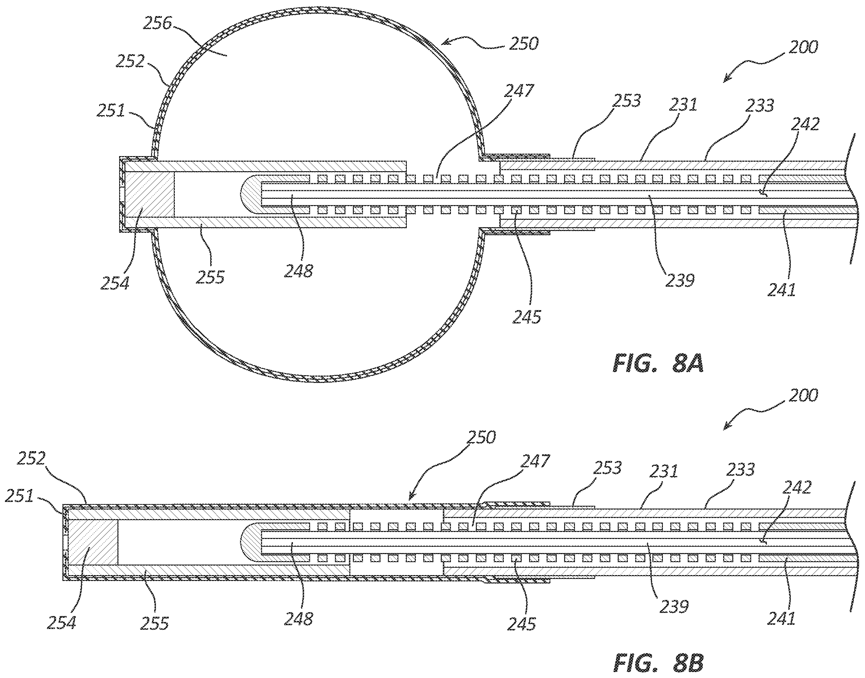

FIGS. 8A-8B illustrate a distal portion of the bone displacement device 200. The distal portion comprises an expandable member 250, a distal portion 233 of the outer tube 231, and a distal portion 245 of the shaft 241. The shaft 241 may extend beyond the distal end of the outer tube 231. According to the invention, the expandable member 250 comprises a balloon 251.

The balloon 251 may comprise a balloon wall, such as a double balloon wall 252 configured to expand radially outward without stretching when air or fluid is injected into the balloon 251 through the annular space 239. A proximal portion of the balloon 251 may be sealingly coupled to the distal end of the outer tube 231. A tie layer 253 may be disposed between the balloon wall 252 and the outer tube 231 to facilitate bonding of the balloon wall 252 to the outer tube 231.

A distal portion of the balloon 251 is sealingly coupled to a distal end of a tip tie tube 255. The tip tie tube 255 extends proximally over the distal portion 245 of the shaft 241. The tip tie tube 255 and shaft 241 is configured in a "piston/cylinder" type arrangements where the shaft 214 is allowed to move with respect to the tip tie tube when the balloon 251 is inflated or deflated. For example, the tip tie tube 255 may be configured to piston proximally over the shaft 241 when the balloon 251 is inflated and to piston distally over the shaft 241 when the balloon 251 is deflated. A plug 254 may be sealingly disposed within the distal end of the tip tie tube 255.

The seal of the proximal end of the balloon 251 to the outer tube 231 and the seal of the distal end of the balloon 251 to the tip tie tube 255 may form a balloon chamber 256 configured to be pressurized. The balloon chamber 256 may be pressurized up to a pressure of about 60 atm. The balloon 251 may be expanded to a diameter of from about 10 mm to about 30 mm.

As shown in FIGS. 8A-8B, the distal portion of the shaft 241 may comprise a plurality of laser cuts 247 (not claimed). The laser cuts 247 may facilitate articulation of the shaft 241 when the tension force is applied to the pull wires 242, as shown in FIG 8B.

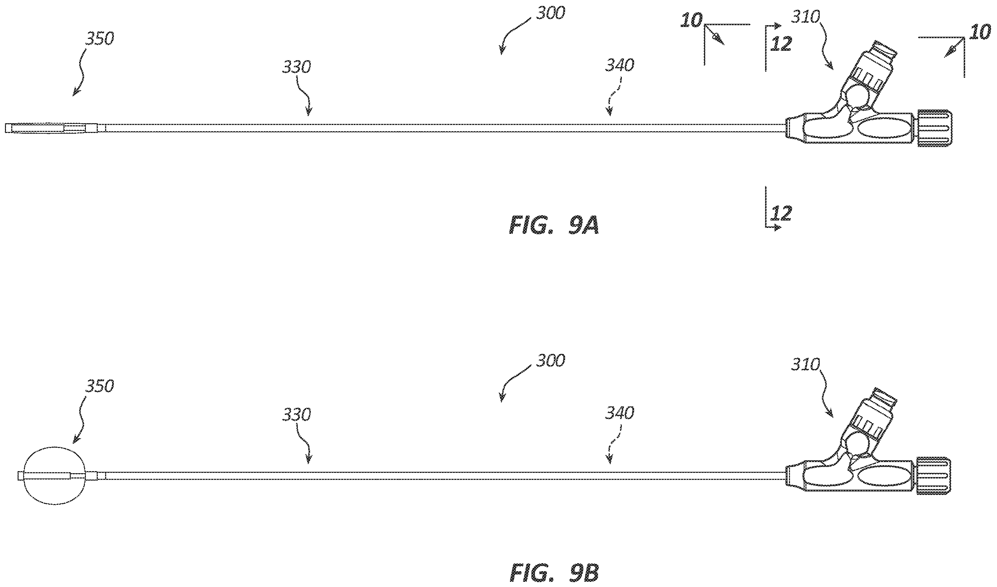

FIGS. 9A-13B depict an example bone displacement device 300. In the illustrated example, the bone displacement device 300 is partially composed of a handle 310, a catheter 330, a stylet 340, and an expandable member 350. FIG. 9A shows the bone displacement device 300 in a ready state where the expandable member 350 is not expanded and a distal portion of the bone displacement device 300 is substantially straight. FIG. 9B shows the bone displacement device 300 in a cavity forming state where the expandable member 350 is expanded and the distal portion is substantially straight.

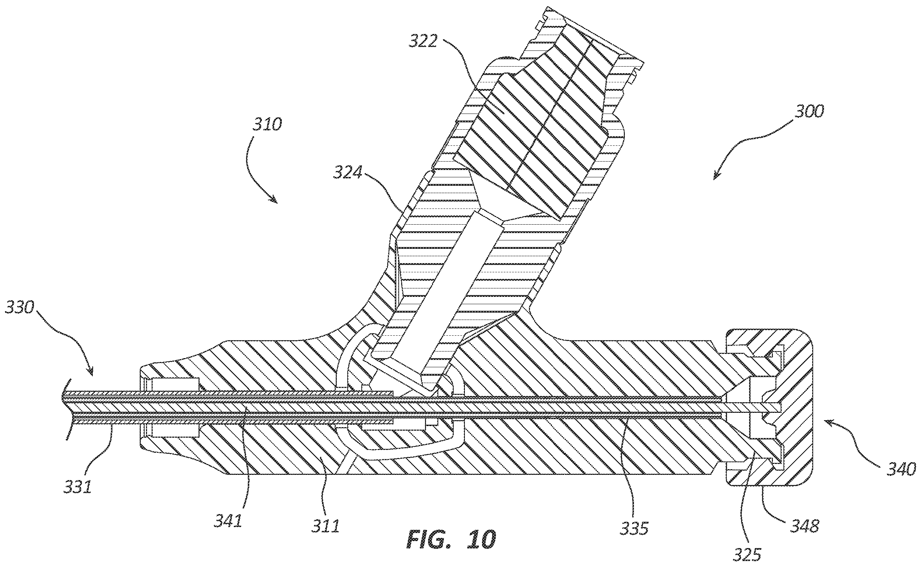

FIG. 10 illustrates a proximal portion of the bone displacement device 300. The proximal portion may comprise the handle 310, a proximal portion of the catheter 330, and a proximal portion of the stylet 340. The catheter 330 may comprise an elongate outer tube 331 and an elongate inner tube 335. A proximal end of outer tube 331 can be coupled to the handle 310. The inner tube 335 may be coaxially disposed within the outer tube 331. A proximal end of the inner tube 335 can be coupled to the handle 310 at a location proximal of the proximal end of the outer tube 331.

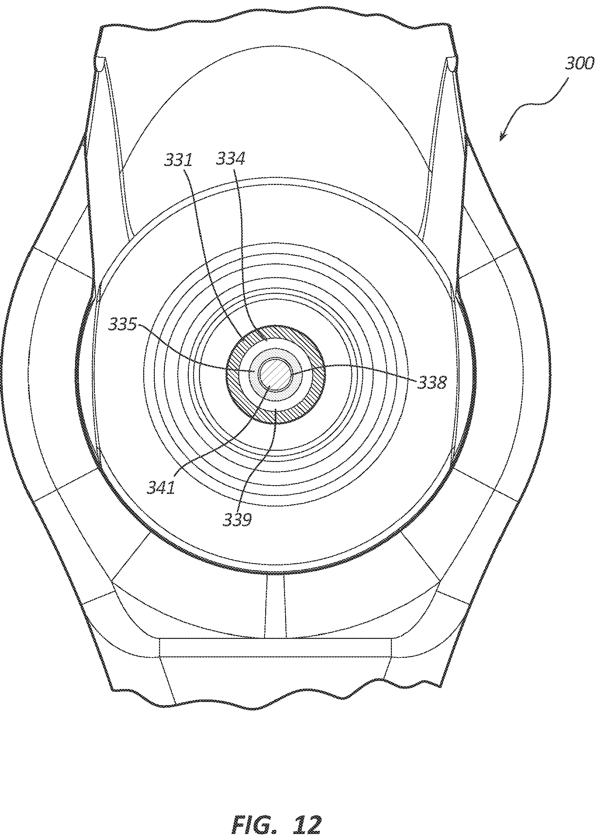

The handle 310 may comprise a body 311. The body 311 may comprise a side port 324 extending laterally from a longitudinal axis of the body 311. The side port 324 may be in fluid communication with an annular space (339 of FIG. 12). A fluid delivery device (e.g., syringe) can be releasably coupled to a proximal portion of the side port 324. A valve member 322 may be disposed within the side port 324. The valve member 322 may be configured to selectively permit air or fluid to be directed through the side port 324, through the annular space 339, and to the expandable member 350 when the fluid delivery device is coupled to the side port 324. The valve member 322 may also be configured to retain the air or fluid within the annular space 339 and the expandable member 350 when the fluid delivery device is removed from the side port 324. The body 311 may include a proximal port 325 in fluid communication with the inner tube 335. The proximal port 325 may be configured to receive the stylet 340. In one example, the proximal port 325 may include a female luer taper and a thread engagement feature.

In the illustrated example, the proximal end of the outer tube 331 can be coupled to the body 311 at a location distal to the side port 324. The inner tube 335 can be coupled to the body 311 at a location proximal to the outer tube 331.

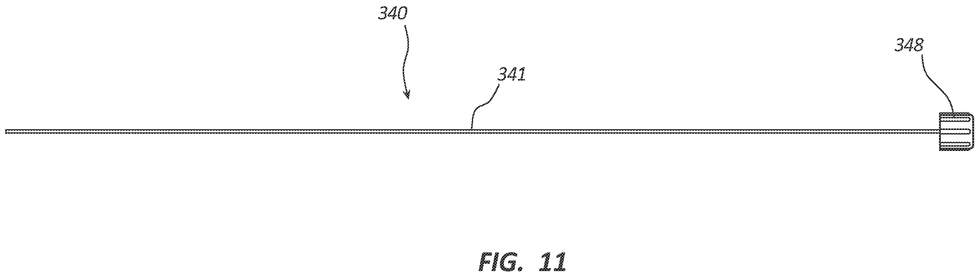

FIG. 11 depicts the stylet 340. The stylet 340 may comprise a shaft 341 and a connector 348. The shaft 341 may be a cylindrical rod. In other examples, the shaft 341 may be a cylindrical tube. A proximal end of the shaft 341 may be fixedly coupled to the connector 348. In the illustrated example, the connector 348 is a male luer nut. The connector 348 may be configured to be removably coupled to the proximal port 325 such that the stylet 340 may be inserted into and removed from the bone displacement device 300 through the proximal port 325.

FIG. 12 illustrates a transverse cross-section of a middle portion of the bone displacement device 300. As illustrated, the outer tube 331 includes an outer lumen 334. The inner tube 335 is shown to be coaxially disposed within the outer lumen 334. An annular space 339 may be defined between the outer tube 331 and the inner tube 335. The inner tube may include an inner lumen 338. The shaft 341 may be coaxially disposed within the inner lumen 338.

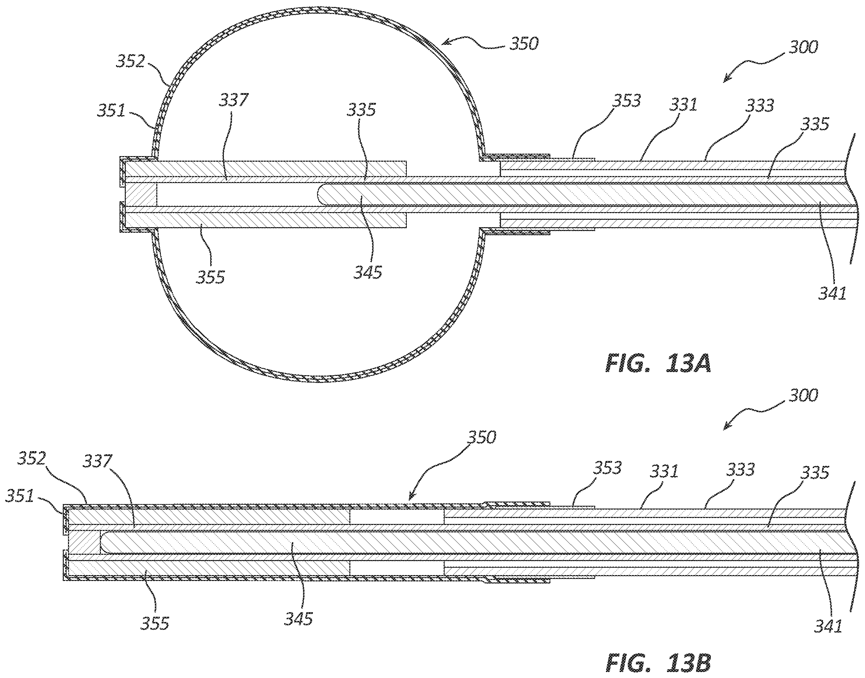

FIGS. 13A-13B illustrate a distal portion of the bone displacement device 300. The distal portion may comprise an expandable member 350, a distal portion 333 of the outer tube 331, a distal portion 337 of the inner tube 335, and a distal portion 345 of the shaft 341. The inner tube 335 and the shaft 341 may extend beyond the distal end of the outer tube 331. The inner tube 335 may extend beyond the distal end of the shaft 341. In the illustrated example, the expandable member 350 comprises a balloon 351.

A proximal portion of the balloon 351 may be sealingly coupled to the distal end of the outer tube 331. A tie layer 353 may be disposed between a balloon wall 352 and the outer tube 331 to facilitate bonding of the balloon wall 352 to the outer tube 331. A distal portion of the balloon 351 may be sealingly coupled to the distal end of the inner tube 335. A tip tie tube 355 may be disposed between the balloon wall 352 and the inner tube 335. The tip tie tube 355 may extend proximally over the distal portion 337 of the inner tube 335 and the distal portion 345 of the shaft 341. The tip tie tube 355 and shaft 341 may be configured to move in a piston/cylinder configuration when the balloon 351 is inflated and deflated. For example, the tip tie tube 335 may move proximally over the shaft 341 when the balloon 351 is inflated, as shown in FIG. 13A, and to piston distally over the shaft 341 when the balloon 351 is deflated, as shown in FIG. 13B.

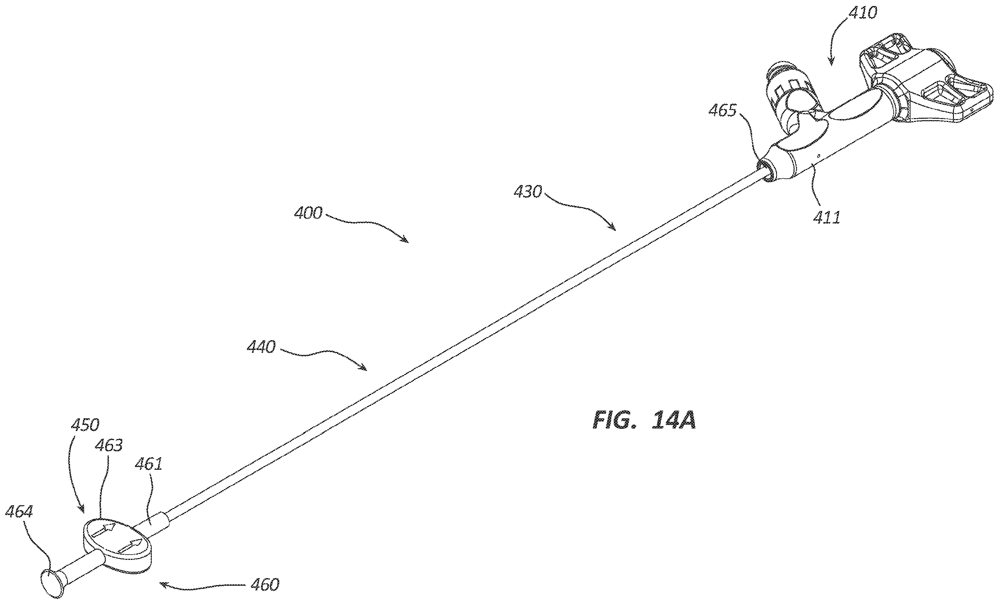

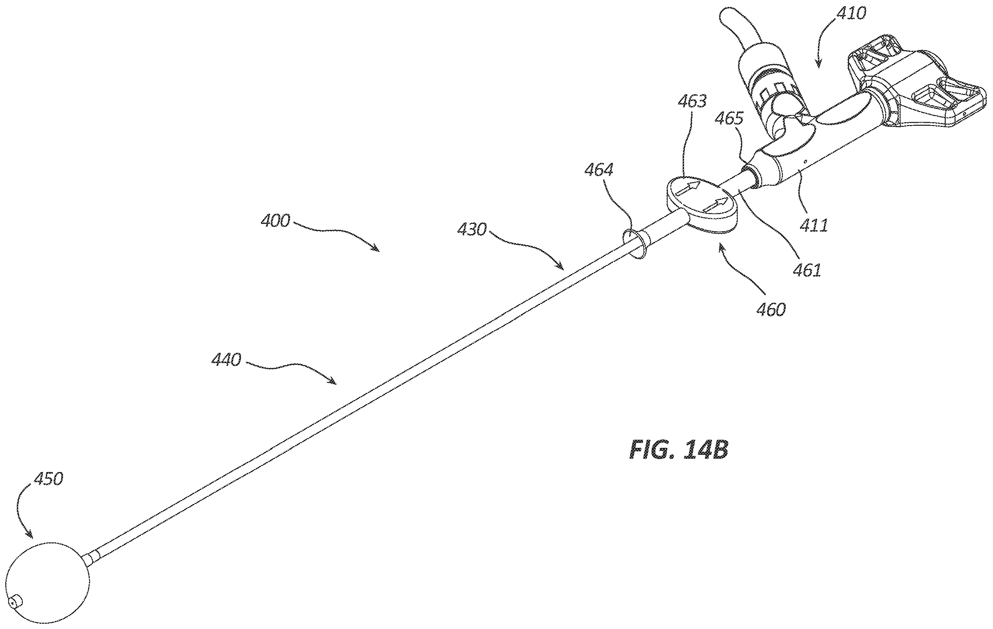

FIGS. 14A-14B depict another embodiment of a bone displacement device 400. In the illustrated embodiment, the bone displacement device 400 comprises a handle 410, a catheter 430, a stylet 440, an expandable member 450, and a protective sleeve 460. FIG. 14A shows the bone displacement device 400 in a package state where the protective sleeve 460 surrounds the expandable member 450. FIG. 14B shows the bone displacement device 400 in a ready state where the protective sleeve 460 is displaced proximally over the catheter 430 and releasably coupled to the handle 410.

In the depicted embodiment, the protective sleeve 460 may include a tubular body 461 and a grip 463 coupled to the tubular body 461. A distal end 464 of the tubular body 461 may be formed in a funnel shape to facilitate passage of the protective sleeve 460 distally over the expandable member 450 when not expanded. A proximal end of the tubular body 461 may be sized to be releasably received into a recess 465 disposed at a distal end of a body 411 of the handle 410, as shown in FIG. 14B. In other embodiments, the proximal end of the tubular body 461 may be sized to fit over a protrusion extending from the distal end of the body 411.

As depicted in the illustrated embodiment, the grip 463 has an oval shape. In other embodiments, the grip 463 may have any suitable shape that is grippable with fingers of a user. For example, the grip 463 may have a rectangular, square, circular, or triangular shape, etc. In some embodiments, the grip 463 may include grip enhancing features, such as ridges, bumps, recesses, etc. In another embodiment, the grip 463 may include indicia (e.g., an arrow) to indicate the direction the protective sleeve 460 could be moved prior to use of the bone displacement device 400.

The protective sleeve 460 may be disposed over the expandable member 450 during a manufacturing assembly of and prior to packaging of the bone displacement device 400. In preparation for a treatment procedure, a user can remove the bone displacement device 400 from its package in the package state, as shown in FIG. 14A, where the protective sleeve 460 is disposed over the expandable member 450. The user may then proximally displace the protective sleeve 460 and releaseably couple the protective sleeve 460 to the handle 410, as shown in FIG. 14B, prior to insertion of the bone displacement device 400 into a vertebral bone of a patient to prevent the protective sleeve 460 from inadvertent distal displacement into a working field . In some procedures, upon removal of the bone displacement device 400 from the vertebral bone, the user may longitudinally distally displace the protective sleeve 460 to rewrap (e.g., radially compress) the expandable member 450 in preparation for re-insertion of the bone displacement device 400.

The scope of the invention is defined by the following claims.

## Claims

1. A bone displacement system (100,200,300,400), comprising:
a bone displacement device (200), comprising:
an elongate outer tube (231);
an elongate stylet (240) coaxially disposed within the outer tube (231), wherein the stylet (240) comprises a tubular shaft (241) with a shaft lumen (243);
a handle (210) coupled to a proximal portion of the outer tube (231) and the stylet (240); and
a balloon (251) disposed adjacent a distal end of the outer tube (231); and
a tip tie tube (255) coupled to a distal end of the balloon (251) and slidably disposed over a distal portion of stylet (240),
wherein the elongate stylet (240) extend partially into the balloon (251) and partially into the tip tie tube (255);
a protective sleeve (460) configured to be disposed around the balloon (251).

2. The bone displacement system (200) of claim 1, wherein the stylet (240) further comprises a plurality of pull wires (242) disposed within the shaft (241).

3. The bone displacement system (200) of claim 1 or claim 2, wherein the protective sleeve (460) comprises a tubular member (461) and a grip (463) coupled to the tubular member (461).

4. The bone displacement system (200) of any one of claims 1-3, wherein the protective sleeve (460) is disposed around the balloon (251) when the bone displacement device (200) is in a package state, and is coupled to the handle (210) when the bone displacement device (200) is in a ready state,
wherein a distal end of the protective sleeve (460) comprises a funnel shape.

5. The bone displacement system (200) of any one of claim 1-4, wherein the tie tip tube (255) comprises a plug (254) at a distal end of the tip tie tube (255).

6. The bone displacement system (200) of claim 5, wherein the tip tie tube (255) is displaced proximally relative to the distal portion of the stylet (240) when the balloon (251) is expanded, and is displaced distally relative to the distal portion of the stylet (240) when the balloon (251) is contracted.

7. The bone displacement system (200) of claim 5 or claim 6, wherein the tip tie tube (255) comprises a braided material.

8. The bone displacement system (200) of any one of claims 1-7, further comprising an elongate inner tube (335) coaxially disposed between the outer tube (231) and the stylet (240).

9. The bone displacement system (200) of claim 8, wherein a proximal portion of the balloon (251) is sealingly coupled to a tie layer (253), and the tie layer (253) is sealingly coupled to a distal portion of the outer tube (231), and wherein a distal portion of the balloon (251) is sealingly coupled to the tip tie tube (255), and the tip tie tube (255) is sealingly coupled to a distal portion of the inner tube (335).

10. The bone displacement system (200) of any one of claims 1-9, wherein a distal end of the balloon (251) and the tip tie tube (255) comprise an opening that align with each other.

11. The bone displacement system (200) of any one of claims 1-10, wherein the handle (210) comprises a side port (324) and a valve member (322) disposed within the side port (324).

12. The bone displacement system (200) of claim 2, wherein the stylet (240) comprises seven pull wires.

13. The bone displacement system (200) of claim 2 or claim 12, wherein the handle (210) comprises a rotatable grip (112), and wherein the plurality of pull wires (242) are coupled to a pull member (116) disposed within the rotatable grip (112).

14. The bone displacement system (200) of claim 13, wherein the pull member (116) comprises male threads configured to engage with female threads of the rotatable grip (112).

15. The bone displacement system (200) of claim 13 or claim 14, wherein the pull member (116) is displaced proximally when the rotatable grip (112) is rotated in a first direction to apply a tension force to the plurality of pull wires (242), and displaced distally when the rotatable grip (112) is rotated in a second direction to release the tension force from the plurality of pull wires (242), and wherein a distal portion of the stylet (240) articulates when the tension force is applied to the plurality of pull wires (242).

## Patentansprüche

1. Knochenverdrängungssystem (100, 200, 300, 400), umfassend:
eine Knochenverdrängungsvorrichtung (200), umfassend:
eine langgestreckte äußere Röhre (231);
einen langgestreckten Mandrin (240), der koaxial in der äußeren Röhre (231) angeordnet ist, wobei der Mandrin (240) einen röhrenförmigen Schaft (241) mit einem Schaftlumen (243) umfasst;
einen Griff (210), der an einen proximalen Abschnitt der äußeren Röhre (231) und des Mandrins (240) gekoppelt ist; und
einen Ballon (251), der einem distalen Ende der äußeren Röhre (231) benachbart angeordnet ist; und
eine Spitzenverbindungsröhre (255), die an ein distales Ende des Ballons (251) gekoppelt ist und gleitfähig über einem distalen Ende des Mandrins (240) angeordnet ist,
wobei sich der langgestreckte Mandrin (240) teilweise in den Ballon (251) und teilweise in die Spitzenverbindungsröhre (255) erstreckt;
eine Schutzhülle (460), die dazu konfiguriert ist, um den Ballon (251) angeordnet zu werden.

2. Knochenverdrängungssystem (200) nach Anspruch 1, wobei der Mandrin (240) ferner eine Vielzahl von in dem Schaft (241) angeordneten Zugdrähten (242) umfasst.

3. Knochenverdrängungssystem (200) nach Anspruch 1 oder Anspruch 2, wobei die Schutzhülle (460) ein röhrenförmiges Element (461) und einen an das röhrenförmige Element (461) gekoppelten Handgriff (463) umfasst.

4. Knochenverdrängungssystem (200) nach einem der Ansprüche 1-3, wobei die Schutzhülle (460) um den Ballon (251) angeordnet ist, wenn sich die Knochenverdrängungsvorrichtung (200) in einem Packungszustand befindet, und an den Griff (210) gekoppelt ist, wenn sich die Knochenverdrängungsvorrichtung (200) in einem Bereitschaftszustand befindet,
wobei ein distales Ende der Schutzhülle (460) eine Trichterform umfasst.

5. Knochenverdrängungssystem (200) nach einem der Ansprüche 1-4, wobei die Spitzenverbindungsröhre (255) einen Stopfen (254) an einem distalen Ende der Spitzenverbindungsröhre (255) umfasst.

6. Knochenverdrängungssystem (200) nach Anspruch 5, wobei die Spitzenverdrängungsröhre (255) relativ zu dem distalen Abschnitt des Mandrins (240) proximal verdrängt wird, wenn der Ballon (251) ausgedehnt wird, und relativ zu dem distalen Abschnitt des Mandrins (240) distal verdrängt wird, wenn der Ballon (251) zusammengezogen wird.

7. Knochenverdrängungssystem (200) nach Anspruch 5 oder Anspruch 6, wobei die Spitzenverbindungsröhre (255) ein geflochtenes Material umfasst.

8. Knochenverdrängungssystem (200) nach einem der Ansprüche 1-7, ferner umfassend eine langgestreckte innere Röhre (335), die koaxial zwischen der äußeren Röhre (231) und dem Mandrin (240) angeordnet ist.

9. Knochenverdrängungssystem (200) nach Anspruch 8, wobei ein proximaler Abschnitt des Ballons (251) dichtend an eine Verbindungsschicht (253) gekoppelt ist und die Verbindungsschicht (253) dichtend an einen distalen Abschnitt der äußeren Röhre (231) gekoppelt ist und wobei ein distaler Abschnitt des Ballons (251) dichtend an die Spitzenverbindungsröhre (255) gekoppelt ist und die Spitzenverbindungsröhre (255) dichtend an einen distalen Abschnitt der inneren Röhre (335) gekoppelt ist.

10. Knochenverdrängungssystem (200) nach einem der Ansprüche 1-9, wobei ein distales Ende des Ballons (251) und der Spitzenverbindungsröhre (255) eine Öffnung umfassen, die sich decken.

11. Knochenverdrängungssystem (200) nach einem der Ansprüche 1-10, wobei der Griff (210) einen seitlichen Port (324) und ein in dem seitlichen Port (324) angeordnetes Ventilelement (322) umfasst.

12. Knochenverdrängungssystem (200) nach Anspruch 2, wobei der Mandrin (240) sieben Zugdrähte umfasst.

13. Knochenverdrängungssystem (200) nach Anspruch 2 oder Anspruch 12, wobei der Griff (210) einen drehbaren Handgriff (112) umfasst und wobei die Vielzahl von Zugdrähten (242) an ein in dem drehbaren Handgriff (112) angeordnetes Zugelement (116) gekoppelt ist.

14. Knochenverdrängungssystem (200) nach Anspruch 13, wobei das Zugelement (116) ein Außengewinde (117) umfasst, das zum Eingriff mit einem Innengewinde (114) des drehbaren Handgriffs (112) konfiguriert ist.

15. Knochenverdrängungssystem (200) nach Anspruch 13 oder Anspruch 14, wobei das Zugelement (116) proximal verdrängt wird, wenn der drehbare Handgriff (112) in einer ersten Richtung gedreht wird, um eine Zugkraft auf die Vielzahl von Zugdrähten (242) auszuüben, und distal verdrängt wird, wenn der drehbare Handgriff (112) in einer zweiten Richtung gedreht wird, um die Zugkraft von der Vielzahl von Zugdrähten (242) freizugeben, und wobei ein distaler Abschnitt des Mandrins (240) gelenkig ausgelenkt wird, wenn die Zugkraft auf die Vielzahl von Zugdrähten (242) ausgeübt wird.

## Revendications

1. Système de déplacement d'os (100, 200, 300, 400), comprenant :
un dispositif de déplacement d'os (200), comprenant :
un tube externe allongé (231) ;
un stylet allongé (240) disposé de manière coaxiale au sein du tube externe (231), dans lequel le stylet (240) comprend une tige tubulaire (241) avec une lumière de tige (243) ;
une poignée (210) couplée à une partie proximale du tube externe (231) et du stylet (240) ; et
un ballonnet (251) disposé adjacent à une extrémité distale du tube externe (231) ; et
un tube de liaison de pointe (255) couplé à une extrémité distale du ballonnet (251) et disposé de manière coulissante sur une partie distale du stylet (240),
dans lequel le stylet allongé (240) s'étend partiellement jusque dans le ballonnet (251) et partiellement dans le tube de liaison de pointe (255) ;
un manchon protecteur (460) configuré pour être disposé autour du ballonnet (251).

2. Système de déplacement d'os (200) selon la revendication 1, dans lequel le stylet (240) comprend en outre une pluralité de fils de traction (242) disposés au sein de la tige (241).

3. Système de déplacement d'os (200) selon la revendication 1 ou 2, dans lequel le manchon protecteur (460) comprend un élément tubulaire (461) et un élément de préhension (463) couplé à l'élément tubulaire (461).

4. Système de déplacement d'os (200) selon l'une quelconque des revendications 1 à 3, dans lequel le manchon protecteur (460) est disposé autour du ballonnet (251) lorsque le dispositif de déplacement d'os (200) est dans un état d'emballage, et est couplé à la poignée (210) lorsque le dispositif de déplacement d'os (200) est dans un état prêt,
dans lequel une extrémité distale du manchon protecteur (460) comprend une forme d'entonnoir.

5. Système de déplacement d'os (200) selon l'une quelconque des revendications 1 à 4, dans lequel le tube de liaison de pointe (255) comprend un bouchon (254) au niveau d'une extrémité distale du tube de liaison de pointe (255).

6. Système de déplacement d'os (200) selon la revendication 5, dans lequel le tube de liaison de pointe (255) est déplacé de manière proximale par rapport à la partie distale du stylet (240) lorsque le ballonnet (251) est déployé, et est déplacé de manière distale par rapport à la partie distale du stylet (240) lorsque le ballonnet (251) est contracté.

7. Système de déplacement d'os (200) selon la revendication 5 ou la revendication 6, dans lequel le tube de liaison de pointe (255) comprend un matériau tressé.

8. Système de déplacement d'os (200) selon l'une quelconque des revendications 1 à 7, comprenant en outre un tube interne allongé (335) disposé de manière coaxiale entre le tube externe (231) et le stylet (240).

9. Système de déplacement d'os (200) selon la revendication 8, dans lequel une partie proximale du ballonnet (251) est couplée de manière étanche à une couche de liaison (253), et la couche de liaison (253) est couplée de manière étanche à une partie distale du tube externe (231), et dans lequel une partie distale du ballonnet (251) est couplée de manière étanche au tube de liaison de pointe (255), et le tube de liaison de pointe (255) est couplé de manière étanche à une partie distale du tube interne (335).

10. Système de déplacement d'os (200) selon l'une quelconque des revendications 1 à 9, dans lequel une extrémité distale du ballonnet (251) et le tube de liaison de pointe (255) comprennent une ouverture qui s'alignent l'une avec l'autre.

11. Système de déplacement d'os (200) selon l'une quelconque des revendications 1 à 10, dans lequel la poignée (210) comprend un orifice latéral (324) et un élément de soupape (322) disposé au sein de l'orifice latéral (324).

12. Système de déplacement d'os (200) selon la revendication 2, dans lequel le stylet (240) comprend sept fils de traction.

13. Système de déplacement d'os (200) selon la revendication 2 ou la revendication 12, dans lequel la poignée (210) comprend un élément de préhension capable de rotation (112), et dans lequel la pluralité de fils de traction (242) sont couplés à un élément de traction (116) disposé au sein de l'élément de préhension capable de rotation (112).

14. Système de déplacement d'os (200) selon la revendication 13, dans lequel l'élément de traction (116) comprend des filets mâles configurés pour entrer en prise avec des filets femelles de l'élément de préhension capable de rotation (112)

15. Système de déplacement d'os (200) selon la revendication 13 ou la revendication 14, dans lequel l'élément de traction (116) est déplacé de manière proximale lorsque l'élément de préhension capable de rotation (112) est tourné dans une première direction pour appliquer une force de tension à la pluralité de fils de traction (242), et déplacé de manière distale lorsque l'élément de préhension capable de rotation (112) est tourné dans une deuxième direction pour libérer la force de tension de la pluralité de fils de traction (242), et dans lequel une partie distale du stylet (240) s'articule lorsque la force de tension est appliquée à la pluralité de fils de traction (242).
